(19) **Europäisches Patentamt / European Patent Office / Office européen des brevets**

(11) **EP 3 673 729 A1**

(12) **EUROPEAN PATENT APPLICATION**
published in accordance with Art. 153(4) EPC

(43) Date of publication:
**01.07.2020 Bulletin 2020/27**

(21) Application number: **18847565.1**

(22) Date of filing: **14.08.2018**

(51) Int Cl.:
**A01H 1/00** *(2006.01)*    **A01G 7/00** *(2006.01)*
**A01G 22/00** *(2018.01)*    **A01H 3/02** *(2006.01)*

(86) International application number:
**PCT/JP2018/030250**

(87) International publication number:
**WO 2019/039349 (28.02.2019 Gazette 2019/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.08.2017 JP 2017159684**

(71) Applicant: **National Agriculture and Food Research Organization**
**Tsukuba-shi, Ibaraki 305-8517 (JP)**

(72) Inventors:
• **KURODA Yosuke**
  **Sapporo-shi**
  **Hokkaido 062-8555 (JP)**

• **TAGUCHI Kazunori**
  **Sapporo-shi**
  **Hokkaido 062-8555 (JP)**
• **OKAZAKI Kazuyuki**
  **Sapporo-shi**
  **Hokkaido 062-8555 (JP)**
• **TAKAHASHI Hiroyuki**
  **Tsukuba-shi**
  **Ibaraki 305-8517 (JP)**
• **KURANOUCHI Toshikazu**
  **Tsukuba-shi**
  **Ibaraki 305-8518 (JP)**

(74) Representative: **Mewburn Ellis LLP**
**Aurora Building**
**Counterslip**
**Bristol BS1 6BX (GB)**

(54) **BEET SELECTION METHOD CAPABLE OF ACCELERATING GENERATION**

(57)    The present invention relates to a technique for accelerating generation advancement of sugar beets that can be used for cultivation aimed at raw material production. The present invention provides a method for selecting sugar beets comprising cultivating sugar beet strains having a chilling requirement under light conditions with a continuous dark period of 6 hours or less per day (preferably, whole-day lighting conditions) without vernalization treatment and selecting a sugar beet strain induced to flower; and a method for breeding sugar beets using the strain selected by the method.

**Description**

[Technical Field]

The present invention relates to a method for selecting a sugar beet capable of accelerated generation advancement.

[Background Art]

Many of crop varieties are bred and developed over a long period of time, which takes at least a decade, so as to achieve "homogeneity" required for variety through genetic fixation over many generations. Biennial crops such as sugar beets have a chilling requirement (vernalization requirement) which prevents plants from flowering unless they undergo a given period of cold temperatures, such as winter, and thus biennial crops take a longer period of time to breed varieties, compared with annual crops such as rice, wheat and its related species, and soybeans.

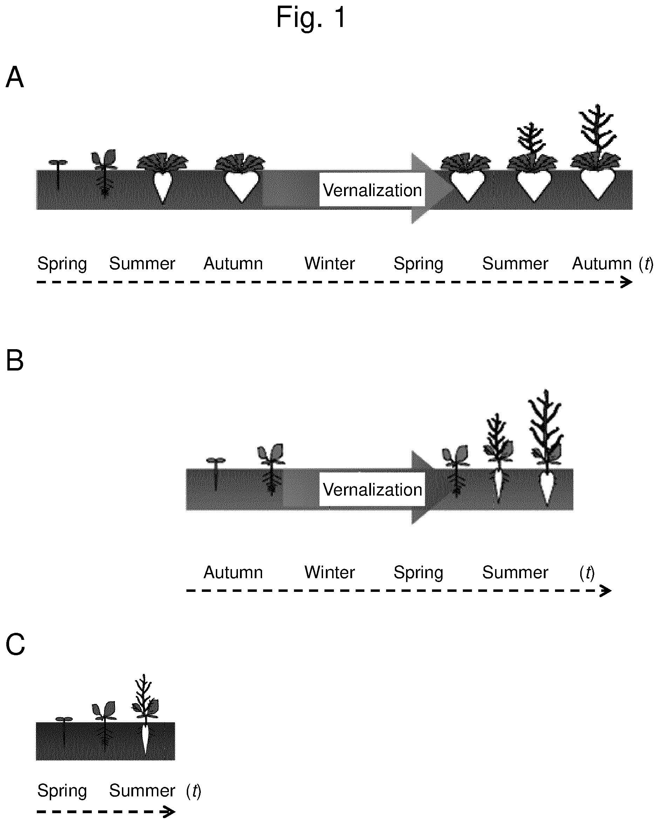

A sugar beet (scientific name: *Beta vulgaris*) is a major raw material of sugar and it is an important agricultural product. Standard sugar beet cultivation methods comprise sowing sugar beet seeds in the spring of the first year, growing them to have enlarged roots (mother roots) grown to a certain size until the autumn of the same year. This period of time is primarily a vegetative growth stage. The vegetatively grown sugar beets are stored at a low temperature of 10°C or lower during the winter for vernalization treatment. When the sugar beets after vernalization treatment are transplanted on the next spring, bolting takes place in the sugar beets; stems elongate and flower buds are formed so that the sugar beets move into a reproductive growth stage. The sugar beets flower and then seeds can be harvested therefrom by the autumn. According to the standard cultivation methods, it takes approximately a year and a half from sowing to seed harvesting (Fig. 1A). In order to speed up the development of sugar beet varieties, a novel technique for accelerated generation advancement of sugar beets is awaited.

A technique for accelerated generation advancement of sugar beets aimed at shortening time to seed harvesting is known. According to the technique, sugar beets are sowed in the autumn and seedlings are grown, and then the seedlings are managed at a low temperature of 10°C or lower for approximately 150 days during the winter for vernalization (Non-Patent Document 1; and Patent Document 1). When transplanting the seedlings on the following spring, bolting and flowering take place and then mature seeds can be harvested by the autumn. According to this technique of accelerated generation advancement, a period from sowing to seed harvesting is shortened to approximately a year (Fig. 1B). Even according to this technique, however, a seed harvesting process requires a long period of time.

While sugar beets are basically biennial, there are some annual sugar beet strains that do not have a chilling requirement (Non-Patent Document 2). Annual sugar beet strains are induced to flower under approximately 16 hour day-length conditions without chilling treatment (Fig. 1C). When outdoor cultivation of annual sugar beet strains is initiated in the spring (late April), which is the common cultivation season in Hokkaido, Japan, accordingly, bolting occurs in June under natural day length conditions (with the day length of approximately 15 hours), flowering is induced, and mature seeds can be harvested by the autumn. According to the technique using annual sugar beet strains, a single seed harvesting process is completed in approximately 4 months after sowing.

Meanwhile, a high yield is also expected for cultivation of sugar beet as a raw material for sugar production. However, bolting significantly decreases the yield and the sugar content of sugar beets. Since annual sugar beet strains undergo bolting and flowering in the outdoor cultivation (under the natural day length conditions), it is difficult to achieve a high yield, and annual sugar beet strains are not suitable for cultivation aimed at production of raw sugar materials.

[Prior Art Documents]

[Patent Documents]

[Patent Document 1] JP 2010-88364 A

[Non-Patent Documents]

Non-Patent Document 1: TAGUCHI, Kazunori, OOGATA, Naoki, and NAKATSUKASA, Keiji, (2004) "Development of method for small-scale accelerated generation advancement by artificial vernalization treatment," Proceedings of the Sugar Beet Research Association, Vol. 46, pp. 23-30
Non-Patent Document 2: Abegg FA, 1936, J. Agric. Res., Vol. 53, No. 7, pp. 493-511

[Summary of the Invention]

[Problem to Be Solved by the Invention]

The problem to be solved by the invention is to provide a technique for accelerated generation advancement of a sugar beet that can be used for cultivation aimed at raw material production.

[Means for Solving the Problem]

As a result of concentrated studies conducted in order to solve the above problem, the present inventors revealed that there is a sugar beet strain having both of the property that enables an inhibition of bolting and flowering for cultivation aimed at raw material production and the property that enables a flowering induction within the shortest period of time and thus an accelerated generation advancement for cultivation aimed at seed harvesting. This has led to the completion of the present invention.

Specifically, the present invention includes the following.

[1] A method for selecting sugar beets, comprising:

cultivating sugar beets having a chilling requirement under light conditions with a continuous dark period of 6 hours or less per day, without vernalization treatment; and
selecting a sugar beet strain induced to flower.

[2] The method for selecting sugar beets according to [1] above comprising:

a) cultivating said sugar beet strains under natural long-day conditions without vernalization treatment and investigating whether or not flowering induction occurs;
b) cultivating said sugar beets under light conditions with a continuous dark period of 6 hours or less per day without vernalization treatment and investigating whether or not flowering induction occurs; and
c) selecting a sugar beet that is not induced to flower in the step a) but is induced to flower in the step b).

[3] The method according to [1] or [2] above, wherein the light conditions with a continuous dark period of 6 hours or less per day are whole-day lighting conditions.
[4] The method according to [2] above, wherein the natural long-day conditions are natural day length conditions with a natural day length of 14 to 17 hours.
[5] The method according to any of [1] to [4] above, wherein whether or not flowering induction occurs is determined based on a bolting rate.
[6] The method for selecting sugar beets according to any of [1] to [5] above, wherein the cultivation under the light conditions with a continuous dark period of 6 hours or less per day is carried out under temperature conditions of 15°C to 30°C.
[7] A method for breeding sugar beets comprising crossing a sugar beet strain selected by the method according to any of [1] to [6] above as a parent strain with another sugar beet strain and obtaining offspring sugar beet plants capable of being induced to flower under light conditions with a continuous dark period of 6 hours or less per day without vernalization treatment.

This description includes the contents as disclosed in Japanese Patent Application No. 2017-159684, on which the present application claims a priority.

[Effects of the Invention]

According to the present invention, a sugar beet having advantageous properties that enable a flowering induction within a short period of time for cultivation aimed at seed harvesting and also enable an inhibition of flowering for cultivation aimed at raw material production can be efficiently selected.

[Brief Description of the Drawings]

[Figure 1] Fig. 1 schematically shows a life cycle of a sugar beet according to conventional cultivation methods. A, standard cultivation; B, conventional method of accelerated generation advancement; C, cultivation of an annual strain.

[Figure 2] Fig. 2 schematically shows typical cultivation and flowering properties of sugar beets selected by the method of the present invention. A, cultivation aimed at raw material production under natural day length conditions; B, cultivation aimed at seed harvesting (accelerated generation advancement) under whole day length conditions.

[Embodiments of the Invention]

Hereafter, the present invention is described in detail.

While the present inventors have advanced the development of a novel technique for accelerated generation advancement of sugar beets, they considered that a sugar beet strain that is suitable for both of "cultivation aimed at seed harvesting" in which flowering within a period of time as short as possible is desired and "cultivation aimed at raw material production" in which flowering is undesired during the cultivation which is usually carried out outdoors would be very useful, if such a strain is available.

The present inventors also considered that there may be cultivation conditions under which a sugar beet strain having a chilling requirement could be induced to flower in non-vernalization or there may be a sugar beet strain that could be induced to flower depending on cultivation conditions, in non-vernalization. The present inventors further considered that a sugar beet strain that does not flower in non-vernalization in the standard outdoor cultivation aimed at raw material production conditions (under natural day length conditions) but is rapidly induced to flower in non-vernalization under artificial long-day conditions with a longer day length than natural day length, if it is present, may enable both "cultivation aimed at raw material production" in which the decrease of yield due to bolting is prevented and "cultivation aimed at seed harvesting" in which seed harvesting (accelerated generation advancement) is completed within a short period of time. The results of test for investigating them showed the presence of a sugar beet strain that is not induced to flower under natural day length conditions in non-vernalization but is induced to flower under whole-day lighting conditions in non-vernalization, as described in Examples below. In addition, some of the sugar beet strains found in the manner described above were found to undergo bolting under light conditions in which a day length would be a slightly shorter than the whole-day lighting conditions. In the present invention, the term "sugar beet strain" refers to a population of sugar beet plant individuals that are genetically homogeneous or almost homogeneous.

The present invention relates to a method for selecting sugar beets comprising cultivating sugar beet strains having a chilling requirement under light conditions with a longer day length (i.e., a shorter dark period) than that under natural long-day conditions (in particular, than that under natural long-day conditions of areas where sugar beets can be cultivated outdoors) without vernalization treatment and selecting a strain induced to flower.

In particular, the present invention relates to a method for selecting sugar beets comprising cultivating sugar beet strains having a chilling requirement under light conditions with a continuous dark period of 6 hours or less per day without vernalization treatment and selecting a strain induced to flower.

In a preferred embodiment, the present invention relates to a method for selecting sugar beets comprising cultivating sugar beet strains having a chilling requirement under whole-day lighting conditions without vernalization treatment and selecting a strain induced to flower.

Since sugar beet (scientific name: *Beta vulgaris*) stores sucrose in the root, it is used as a raw material for sugar production as with sugar cane. Sugar beets are cold-climate crops and are cultivated mainly in temperate to subarctic zones. Sugar beets are basically biennial. The biennial plants are required to be exposed to low temperature for a given period of time to induce the flowering ability; that is, the biennial plants have a chilling requirement. The expression "sugar beets have/having a chilling requirement" in the context of the present invention means that the sugar beets need to be exposed to a low temperature of 10°C or lower (preferably 0°C to 10°C; or preferably 4°C to 7°C in case of artificial low temperature treatment) for a given period of time (depending on varieties, but of typically 40 to 150 days) to induce flowering under natural day length in areas where sugar beets can be cultivated outdoors. Such treatment at low temperature is generally referred to as "vernalization treatment" (or vernalization).

The terms "in non-vernalization," "without vernalization treatment," and "with no vernalization treatment" used in the context of the present invention mean that a vernalization treatment is not applied to sugar beets, and more specifically, not exposing sugar beets to a temperature or a time that causes vernalization. Typically, the term refers to that not exposing sugar beets to a low temperature of 10°C or lower for more than 40 days. The expression "low temperature of 10°C or lower" means that the daily average temperature is 10°C or lower. The daily average temperature is an average of 24 observed values of temperatures at every hour on the hour from 1:00 (1:00 a.m.) to 24:00 (12:00 a.m.) of a day. In the present invention, the state without vernalization treatment is referred to as "non-vernalization."

The term " light conditions with a continuous dark period of 6 hours or less per day" used in the present invention means a light-dark cycle with a continuous dark period of 6 hours or less per day. It is known that flowering induction of plants is more influenced by the length of a continuous dark period rather than the day length (i.e., a light period) itself. It is considered necessary that a dark period continues without interruption in principle. The "light conditions with a continuous dark period of 6 hours or less per day" basically correspond to light conditions with a light period (day length) of 18 hours or longer per day. The light conditions with a continuous dark period of 6 hours or less per day may have, for example, a continuous dark period of 5.5 hours or less, 5 hours or less, 4.5 hours or less, 4 hours or less, 3.5 hours or less, 3 hours or less, 2.5 hours or less, 2 hours or less, 1.5 hours or less, or 1 hour or less per day; or may be whole-day lighting conditions. A light period and a dark period in a day may be each the same or different from day to day. In the present invention, the dark period refers to a duration in darkness or under light conditions with a little light such that flowering is not induced (typically with a illuminance of lower than 2 1x).

The term "whole-day lighting conditions" used in the context of the present invention means cultivation conditions having a day length (a light period) of approximately 24 hours (23.5 hours to a whole day) per day. The "whole-day lighting conditions" have little dark period (a dark period of 30 minutes or shorter) or have no dark period (a dark period of 0 minutes; i.e., 24-hour day length). The term "day length" or "light period" used herein refers to a duration during which a sugar beet is exposed to a light level such that flowering can be induced.

In the present invention, a type of light generating the light conditions, including whole-day lighting conditions, is not particularly limited. The light may be sunlight, artificial illumination, or a combination of sunlight with artificial illumination. Cultivation of sugar beet strains under the light conditions with a continuous dark period of 6 hours or less per day may be carried out under natural day length conditions with night supplemental lighting using artificial illumination. Artificial illumination may be any lighting by an incandescent lamp, fluorescent lamp, LED, or the like. In the present invention, the illuminance on a sugar beet lighted by artificial illumination is preferably 10 lx or more, and more preferably approximately 50 to 1,000 lx. The illuminance using an incandescent lamp may be, but not limited to, for example, 100 to 500 lx. Under the whole-day lighting conditions, the flowering rate tends to be increased and the period to flowering induction tends to be shortened when using the combination of sunlight (approximately 100,000 lx at maximum) during daytime and artificial illumination at night, compared with using artificial illumination (approximately 1,000 lx at maximum) throughout the day.

Under the light conditions with a continuous dark period of 6 hours or less per day, preferably, sugar beet strains are cultivated under temperature conditions of a temperature of typically from more than 10°C to 40°C, and preferably from 15°C to 30°C (atmospheric temperature or room temperature). However, the temperature conditions are not limited thereto, provided that plants are not vernalized.

Under the light conditions with a continuous dark period of 6 hours or less per day (long-day conditions), such as under the whole-day lighting conditions, the cultivation of sugar beets may be carried out for a period of time and in the stage necessary for flower-bud formation and flowering induction of sugar beets. The cultivation period varies depending on environmental factors such as light intensity, and it is, but not limited to, preferably approximately 40 days or longer (at least 35 days, for example, 35 to 80 days). Sugar beets may be exposed to such light conditions from the sowing stage or immediately after germination; alternatively, sugar beets may be exposed to such light conditions after they grow to a certain extent. When sugar beets are grown under the light conditions for typically approximately 40 days, flower-bud formation is initiated and bolting occurs. Flowering begins 2 or 3 weeks thereafter, and flowering continues up to the timing of initial seed harvesting (approximately 4 months after sowing).

Sugar beet strains having a chilling requirement are cultivated under the light conditions with a continuous dark period of 6 hours or less per day (more preferably under the whole-day lighting conditions) without vernalization treatment as described above, thereby leading to the observation of flowering induction in some particular sugar beet strains. The particular sugar beet strains bolt approximately 1 to 3 months after sowing, followed by flowering. The term "bolting" refers to an elongation of a flower stem followed by a formation of a flower bud. Sugar beet strains are investigated as to whether or not bolting occurred in each of plant individuals, and a bolting rate of each sugar beet strain can be determined in accordance with the following equation.

$$\text{Bolting rate (\%)} = \text{the number of plant individuals that have bolted /the number of all plant individuals x 100}$$

Whether or not flowering induction occurs in a sugar beet strain can be determined based on the bolting rate. In the context of the present invention, if the bolting rate of a sugar beet strain is 80% or higher (ideally 100%), the sugar beet strain is determined to be induced to flower under the cultivation conditions adopted. If the bolting rate is 20% or lower (ideally 0%), the sugar beet strain is determined to be not induced to flower under the cultivation conditions adopted. If the bolting rate is more than 20% to lower than 80%, it is considered that the sugar beet strain may lack genetic homogeneity, or may have variations between individuals because of the dark period used being around the length of the critical dark period.

Determination as to whether or not flowering induction occurs may be performed based on the bolting rate as described above, or alternatively may be performed in accordance with other criteria. For example, the determination may be performed based on the proportion of the number of flowered individuals relative to the number of all plant individuals, instead of the bolting rate, on the same threshold as the determination based on the bolting rate.

In the manner described above, sugar beet strains that have been induced to flower under the cultivation conditions described above can be selected from among sugar beet strains having a chilling requirement. The selected sugar beet strains have advantageous properties such that they do not flower under natural day length conditions (in particular, natural day length conditions of areas where sugar beets can be cultivated outdoors) without vernalization treatment but they are induced to flower under light conditions with a shorter dark period, for example, light conditions with a continuous dark period of 6 hours or less per day (preferably under whole-day lighting conditions) without vernalization treatment.

In the method of the present invention, it is also preferred that test sugar beet strains be cultivated, without vernalization treatment, under natural long-day conditions and investigated for whether or not flowering induction occurs. It is more preferred that sugar beet strains be cultivated under natural long-day conditions of an area where the sugar beet strains are to be cultivated and investigated for whether or not flowering induction occurs. From the investigation results, whether or not the test sugar beet strains are induced to flower without vernalization treatment under the natural day length conditions of the area of interest can be examined. That is, whether or not the test sugar beet strains have a chilling requirement under such natural day length conditions can be determined or examined.

In one embodiment of the present invention, the method for selecting sugar beets comprises:

a) cultivating the sugar beet strains under natural long-day conditions without vernalization treatment and investigating whether or not the flowering induction occurs;
b) cultivating the sugar beets under light conditions with a continuous dark period of 6 hours or less per day (preferably under whole-day lighting conditions) without vernalization treatment and investigating whether or not flowering induction occurs; and
c) selecting a sugar beet that is not induced to flower in the step a) but is not induced to flower in the step b). According to this method, sugar beets that are suitable for both cultivation aimed at raw material production and cultivation aimed at seed harvesting can be efficiently obtained.

The term "natural long-day conditions" refers to natural day length conditions (natural photoperiodic conditions) with a natural day length, i.e., a duration from the time of sunrise to the time of sunset (the daytime length), that is longer than a duration from the time of sunset to the time of sunrise (the night length). The "natural long-day conditions" is preferably natural day length conditions in the summer of an area where sugar beets can be cultivated outdoors (typically the temperate to subarctic zones or areas with a latitude of 30 to 60 degrees). The natural long-day conditions are, but not limited to, natural long-day conditions having a natural day length (a daytime length) of typically 14 to 17 hours, preferably 15 to 17 hours, and more preferably 15 to 16 hours per day. Under the natural long-day conditions, the night length (a duration from the time of sunset to the time of sunrise) is typically 7 to 10 hours, preferably 7 to 9 hours, and more preferably 8 to 9 hours per day. The natural day length of the natural day length conditions may also be referred to simply as a "day length," if it is clear from the context of the present invention. Under natural long-day conditions, sugar beets can be cultivated typically in an area at a latitude of 30 to 60 degrees, and preferably at a latitude of 35 to 55 degrees, 35 to 50 degrees, or 30 to 50 degrees of latitude, for example, an area at a latitude of 40 to 45 degrees, including Hokkaido, Japan.

Cultivation under natural long-day conditions (natural day length conditions) may be carried out for a period of time and in the stage necessary for flower-bud formation and flowering induction of sugar beets. The cultivation period varies depending on environmental factors such as light intensity, and it is preferred that the cultivation period is, but not limited to, typically approximately 40 days or longer (for example, 35 to 80 days). The cultivation period under the natural long-day conditions can include a day at which the natural day length reaches a maximum (i.e., the day of the summer solstice). Sugar beets may be exposed to such conditions from the sowing stage or immediately after germination; alternatively, sugar beets may be exposed to such conditions after they grow to a certain extent. Under the natural long-day conditions, sugar beets are cultivated under temperature conditions of a temperature of typically from more than 10°C to 40°C, and preferably from 15°C to 30°C (atmospheric temperature or room temperature). However, the temperature conditions are not limited thereto, provided that plants are not vernalized.

Under the natural long-day conditions described above, annual sugar beet strains generally flower without vernalization treatment; however, sugar beet strains selected by the method of the present invention do not flower without vernalization treatment. The occurrence of flowering induction can be determined in accordance with the criteria as described above.

It is preferred that the same sugar beet strains as those cultivated under the natural long-day conditions described above be cultivated, without vernalization treatment, under light conditions with a continuous dark period of 6 hours or less per day (more preferably under whole-day lighting conditions) and be investigated as to whether or not flowering induction occurs. Cultivation under such light conditions and determination about flowering induction are as described above. The light conditions with a continuous dark period of 6 hours or less per day preferably involve a continuous dark period that is preferably shorter by at least 2 hours, further preferably by at least 3 hours, and particularly preferably by at least 4 hours, compared with the night length of the day of the summer solstice of an area where test sugar beet strains are to be cultivated.

In the present invention, sugar beets may be cultivated outdoors or indoors. Outdoor cultivation can be carried out, for example, in the field. Indoor cultivation can be carried out in, for example, a greenhouse such as a glass or plastic greenhouse, a plant factory, a clean room, a laboratory, an incubator, or an artificial climate chamber. While the cultivation under natural long-day conditions may be preferably carried out outdoors, the cultivation may be carried out in a facility which enables a cultivation under natural day length conditions, such as a sunlight permeable glass greenhouse or plastic greenhouse. The cultivation under light conditions with a continuous dark period of 6 hours or less per day may be preferably carried out indoors, but it may be carried out outdoors with lighting facilities. Raising of seedlings of sugar beets after sowing may be managed indoors or outdoors. For example, the raising of seedlings after sowing may be managed indoors, and the seedlings may then be transferred to and cultivated in the outdoor field. Alternatively, the raising of seedlings after sowing may be managed outdoors, and the seedlings may then be transferred to and cultivated in the indoor facility, such as a greenhouse. The cultivation may be carried out either outdoors or indoors from the stage of the management of the raising of seedlings after sowing.

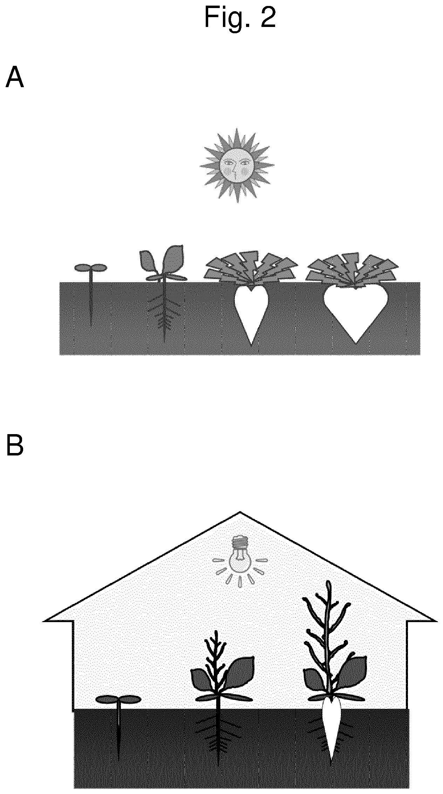

The method of the present invention enables efficient selection of day length-sensitive sugar beet strains (e.g., Fig. 2) that do not flower under natural day length conditions equivalent or similar to natural day length conditions of an area where sugar beets can be cultivated outdoors, and to, in particular, the natural long-day conditions tested in the step a) above, without vernalization treatment; but are induced to flower in non-vernalization under light conditions with a shorter dark period, for example, light conditions with a continuous dark period of 6 hours or less per day (e.g., the light conditions tested in the step b), and more preferably whole-day lighting conditions). Such sugar beet strains can be cultivated without bolting when they are not subjected to vernalization treatment under natural day length conditions. This can reduce the decrease of a yield or sugar content caused by bolting in the cultivation aimed at raw material production. On the other hand, the sugar beet strains can be induced to flower in non-vernalization under light conditions with a continuous dark period of 6 hours or less per day (more preferably under whole-day lighting conditions) and can produce seeds within approximately 4 months. Thus, accelerated generation advancement can be realized in cultivation aimed at seed harvesting; for example, three generations can be produced in a year. In the present invention, sugar beet strains obtainable by the selection method (i.e., day length-sensitive sugar beet strains) are also referred to as BLOND strains.

The sugar beet strains selected by the method according to the present invention preferably have the unique flowering properties as dominant traits. The sugar beet strains selected by the method according to the present invention can also be used as a parent strain for introducing the flowering properties into other sugar beet plants.

Specifically, the present invention provides a method for breeding sugar beets comprising crossing a sugar beet strain selected by the method according to the present invention as a parent strain with another sugar beet strain and obtaining offspring sugar beet plants. Preferred offspring sugar beet plants obtained by the method can be induced to flower without vernalization treatment, for example, via cultivation under light conditions with a continuous dark period of 6 hours or less per day, and more preferably under whole-day lighting conditions.

The another sugar beet strain to be crossed with the selected sugar beet strain may be an annual or biennial strain. Offspring plants obtained by the crossing can inherit the flowering properties (i.e., the ability to be induced to flower, without vernalization treatment, under a short dark period or under whole-day lighting conditions) of the sugar beet strains obtained by the selection method according to the present invention, preferably as dominant traits. By repeating crossing, subsequent generations, such as F1 seeds, F2 seeds, and F3 seeds, may further be obtained as offspring plants. The offspring plants may be homozygous or heterozygous for a responsible gene for the flowering properties. Preferably, the offspring plants obtained via crossing may be verified to be induced to flower, without vernalization treatment, under light conditions with a continuous dark period of 6 hours or less per day, and more preferably under whole-day lighting conditions, by the method described above. Also preferably, the offspring plants obtained via crossing may be cultivated under natural long-day conditions in which annual sugar beet strains are generally induced to flower and then they may be verified not to be induced to flower. Based on these verifications, offspring sugar beet plants that are induced to flower under light conditions with a continuous dark period of 6 hours or less per day (preferably under whole-day lighting conditions) without vernalization treatment can be selected and obtained. By the method of breeding according to the present invention, day length-sensitive flowering properties such that plants do not flower without vernalization treatment under natural day length conditions of an area where sugar beets can be cultivated outdoors but are induced to flower, without vernalization treatment, under light conditions with a shorter dark period, such as light conditions with a continuous dark period of 6 hours or less per day (more preferably whole-day lighting conditions), can be introduced into various sugar beet strains.

[Examples]

Hereafter, the present invention is described in greater detail with reference to Examples, although the technical scope of the present invention is not limited to Examples.

[Example 1]

1. Summer cultivation test (first year)

Various genetic resource sugar beet strains owned by Hokkaido Agricultural Research Center, National Agriculture and Food Research Organization, Japan were cultivated in the test fields (Memuro-cho, Kasai-gun, Hokkaido, Japan) from April to October outdoors and in a greenhouse. Each strain was investigated for the occurrence of bolting, which is a morphological change observed when flowering induction is initiated, and flowering properties. For comparison, a sugar beet annual strain TK 76-49/2mm-O and a sugar beet biennial strain TA-33BB-O, as type strains, were cultivated in the same manner and investigated.

a) Outdoor field cultivation

Sugar beets were sowed in paper pots on April 18. After sowing, the raising of seedlings was managed outdoors. The seedlings were grown to have 2 to 4 leaves and then transplanted into the field. The date of transplanting into the field was May 18. The transplanted sugar beets were grown outdoors under the natural day length conditions. Thereafter, the number of plant individuals that have bolted up to September 1 was counted and the bolting rate was calculated in accordance with the following equation.

$$\text{Bolting rate (\%)} = \text{the number of plant individuals that have bolted /the number of all plant individuals} \times 100$$

b) Glass greenhouse cultivation

Sugar beets were sowed on April 18. After sowing, the raising of seedlings was managed in a glass greenhouse under whole-day lighting conditions (cultivation under natural day length with lighting at night; night lighting conditions: incandescent lamp 200 W, 300 lx, from 18:00 (6:00 p.m.) to 5:00 (5:00 a.m.) on the following morning) under temperature conditions of 24°C on average (18°C to 30°C). The seedlings were grown to have 2 to 4 leaves and then transplanted into pots (diameter: 10 cm; height: 20 cm). The date of transplanting was May 18. Thereafter, the seedlings were grown in the glass greenhouse under the same whole-day lighting conditions and temperature conditions as described above. The number of plants that have bolted up to June 17 was counted, and the bolting rate was calculated in accordance with the equation described in Section a) above. On April 18, the time of sunrise was 4:42 (4:42 a.m.) and the time of sunset was 18:13 (6:13 p.m.) in Memuro-cho. On June 17, the time of sunrise was 3:48 (3:48 a.m.) and the time of sunset was 19:09 (7:09 p.m.). In Memuro-cho, the natural day length of the day of the summer solstice of this year (June 23) was 15 hours and 22 minutes.

As a result of the cultivation test described above, two sugar beet strains, i.e., NK-420 and NK-422, were found to be rarely induced to flower in the outdoor field cultivation and to be induced to flower without the vernalization period in the cultivation under whole-day lighting conditions in a glass greenhouse. These strains were designated as BLOND (bolting by longer than natural day length) strains.

In the outdoor field cultivation (summer cultivation from April to October, the natural day length), specifically, the bolting rate of the annual strain TK 76-49/2mm-O and that of the biennial strain TA-33BB-O were 100% and 0%, respectively. In contrast, the BLOND strains were rarely induced to flower and continued vegetative growth (the bolting rate of 0% or 11%) in the cultivation, and the frequency of flowering induction was very low as with the biennial strain TA-33BB-O (Table 1). Flowering properties of the BLOND strains were distinctly different from those of the annual strain TK 76-49/2mm-O.

Under the whole-day lighting conditions (with the day length of 24 hours) in the glass greenhouse, the bolting rate of the annual strain TK 76-49/2mm-O and that of the biennial strain TA-33BB-O were 100% and 0%, respectively, as with the case of outdoor field cultivation. In contrast, the BLOND strains exhibited the bolting rate of 100% under the conditions, as with the annual strain TK 76-49/2mm-O. Therefore, it was shown that the BLOND strains were induced to flower, without vernalization, under whole-day lighting conditions (Table 1).

The BLOND strain (NK-420 or NK-422) was crossed with the sugar beet biennial strain NK-377 (Hokkaido Agricultural Research Center, National Agriculture and Food Research Organization), thereby obtaining first filial generation (F1 strains; NK-377 x NK-420; or NK-377 x NK-422).

2. Summer cultivation test (second year)

The BLOND strains were subjected to the cultivation test on the following year as described below and investigated for the flowering properties. In addition, F1 strains (NK-377 x NK-420 and NK-377 x NK-422), F1 type strains (NK-377mm-CMS (biennial) x TA-33BB-O (annual)), and the annual strain TA-33BB-O and the biennial strain Monohikari as type strains were subjected to the cultivation test in the same manner.

c) Outdoor field cultivation

Sugar beets were sowed on April 9. After sowing, the raising of seedlings was managed in a glass greenhouse under natural day length conditions and temperature conditions such that the indoor temperature (the lowest temperature) was gradually lowered from approximately 20°C to 10°C so as to prevent spindly growth. The seedlings were grown to have 2 to 4 leaves and then transplanted into the field. The date of transplanting into the field was May 9. The transplanted sugar beets were grown outdoors under natural day length. Thereafter, the number of plant individuals that have bolted up to August 21 was counted, and the bolting rate was calculated in the same manner as in Section 1. a) above.

d) Glass greenhouse cultivation

Sugar beets were sowed on April 9. After sowing, the raising of seedlings was managed in a glass greenhouse under whole-day lighting conditions (cultivation under natural day length with lighting at night; night lighting conditions: incandescent lamp 200 W, 300 lx, from 18:00 (6:00 p.m.) to 5:00 (5:00 a.m.) on the following morning) under temperature conditions of 24°C on average (18°C to 30°C). The seedlings were grown to have 2 to 4 leaves and then transplanted into pots (diameter: 10 cm; height: 20 cm). The date of transplanting was May 8. Thereafter, the seedlings were grown in the glass greenhouse under the same whole-day lighting conditions and temperature conditions as described above. The number of plant individuals that have bolted up to June 11 was counted, and the bolting rate was calculated in the same manner as in Section 1. a) above.

The bolting rates of the BLOND strains (NK-420 and NK-422) were found to be similar to those of the first year. The bolting rates were 0% for the both strains under the natural day length conditions, and the bolting rate of the NK-420 strain was 100% and that of the NK-422 strain was 84% under the whole-day lighting conditions. The bolting rates were 0% for the both F1 strains (NK-377 x NK-420, and NK-377 x NK-422) under the natural day length conditions and the bolting rate of the NK-377 x NK-420 strain and that of the NK-377 x NK-422 strain were 100% and 87%, respectively, under the whole-day lighting conditions, which were similar to the bolting rates of the BLOND strains (Table 1). The bolting rate of the annual strain TA-33BB-O and that of the F1 type strain were 100% both under the natural day length conditions and the whole-day lighting conditions. In contrast, the bolting rate of the biennial strain Monohikari was 0% both under the natural day length conditions and the whole-day lighting conditions. The F1 type strain obtained by crossing between the annual strain (dominant traits) and the biennial strain (recessive traits) exhibited the bolting rate of 100% both under the natural day length conditions and the whole-day lighting conditions. That is, the BLOND strains were distinctly different from the F1 type strains in terms of flowering properties.

These results indicate the presence of sugar beet strains with unique flowering properties such that they do not flower under natural day length conditions but are induced to flower under whole-day lighting conditions. Flowering properties of the F1 strains obtained by crossing between the BLOND strains and the biennial strain NK-377 were the same as those of the BLOND strains. This indicates that flowering properties of the BLOND strains are dominantly inherited to the offspring as with the case of the annual strain TA-33BB-O.

Table 3 shows the number of days up to bolting in the glass greenhouse in the summer cultivation test. The number of days up to bolting for the BLOND strains and the F1 strains were similar to those for the annual strain.

$$\text{The number of days up to bolting} = \text{the total number of days from sowing to the initiation of bolting for strains that have bolted} / \text{the total number of strains that have bolted}$$

F1 strains (NK-377 x NK-420, and NK-377 x NK-422) were crossed with the biennial strain NK-310 as a recurrent parent to obtain first backcross generation (BC1 strains; NK-377 x NK-420 x NK-310 and NK-377 x NK-422 x NK-310).

Additionally, the BLOND strain (NK-420 or NK-422) was crossed with the sugar beet biennial strain NK-310 (Hokkaido Agricultural Research Center, National Agriculture and Food Research Organization) to obtain second filial generation (F2 strains; NK-310 x NK-420 and NK-310 x NK-422).

3. Summer cultivation test (third year)

The BLOND strains were subjected to the cultivation test on the third year in the same manner to investigate the flowering properties. Also, the F2 strains (NK-310 x NK-420, and NK-310 x NK-422), the F2 strains (NK-310 x NK-420, and NK-310 x NK-422), the BC1 strains (NK-377 x NK-420 x NK-310, and NK-377 x NK-422 x NK-310), the F1 type strain (NK-377mm-CMS (biennial) x TA-33BB-O (annual)), the annual strain TA-33BB-O and the biennial strain NK-377mm-O as type strains, and the first backcross generation as a type strain (BC1 type strain; NK-377mm-CMS x TA-33BB-O x NK-310mm-O) were subjected to the cultivation test in the same manner. NK-310mm is substantially genetically equivalent to NK-377mm, and thus such crossing with NK-310mm falls into the backcross category.

1) Outdoor field cultivation (third year)

Sugar beets were sowed on April 9. After sowing, the raising of seedlings was managed in a glass greenhouse under natural day length conditions and temperature conditions such that the indoor temperature (the lowest temperature) was gradually lowered from approximately 20°C to 10°C. The seedlings were grown to have 2 to 4 leaves and then transplanted into the field. The date of transplanting was May 13. The transplanted sugar beets were grown outdoors under natural day length conditions. Thereafter, the number of plant individuals that have bolted up to August 26 was counted, and the bolting rate was calculated in the same manner as in Section 1. a) above.

2) Glass greenhouse cultivation (third year)

Sugar beets were sowed on April 21. After sowing, the raising of seedlings was managed in a glass greenhouse under whole-day lighting conditions (cultivation under natural day length with lighting at night; night lighting conditions: incandescent lamp 200 W, 300 lx, from 18:00 (6:00 p.m.) to 5:00 (5:00 a.m.) on the following morning) under temperature conditions of 24°C on average (18°C to 30°C). The seedlings were grown to have 2 to 4 leaves and then transplanted into pots (diameter: 10 cm; height: 20 cm). The date of transplanting was May 15. Thereafter, the seedlings were grown in the glass greenhouse under the same whole-day lighting conditions and temperature conditions as described above. The number of plant individuals that have bolted up to June 30 was counted, and the bolting rate was calculated in the same manner as in Section 1. a) above.

The bolting rates of the BLOND strains (NK-420 and NK-422), the annual strain, and the biennial strain were found to be similar to those of the first year and the second year. Both the F2 strains (NK-310 x NK-420, and NK-310 x NK-422) obtained by crossing between the BLOND strains and the biennial strain exhibited the bolting rate of 0% under the natural day length conditions, and the bolting rate of the NK-310 x NK-420 strain and that of the NK-310 x NK-422 strain were 83% and 98%, respectively, under the whole-day lighting conditions (Table 1). Both the BC1 strains (NK-377 x NK-420 x NK-310, and NK-377 x NK-422 x NK-310) obtained by crossing the BLOND strains with the biennial strain as a recurrent parent exhibited the bolting rate of 0% under the natural day length conditions and the bolting rate of the NK-377 x NK-420 x NK-310 strain and that of the NK-377 x NK-422 x NK-310 strain were 55% and 48%, respectively, under the whole-day lighting conditions (Table 1). The bolting rate of the BC1 type strain (NK-377mm-CMS x TA-33BB-O x NK-310mm-O) was 44% (Table 1).

From the flowering properties of the F2 strains, and the flowering properties of the F1 strains shown in the cultivation test on the second year, it was shown that flowering properties of the BLOND strains were attributed to a small number of genes with strong effects.

[Table 1]
Flowering properties shown in summer cultivation test

| Sugar beet strain name | Outdoor field (natural day length) | | | | | | Glass greenhouse (whole-day lighting) | | | | | |
|---|---|---|---|---|---|---|---|---|---|---|---|---|
| | 1st year | | 2nd year | | 3rd year | | 1st year | | 2nd year | | 3rd year | |
| | N | Bolt (%) | N | Bolt (%) | N | Bolt (%) | N | Bolt (%) | N | Bolt (%) | N | Bolt (%) |
| NK-420 | 9 | 11 | 11 | 0 | 42 | 0 | 2 | 100 | 8 | 100 | 12 | 100 |
| NK-422 | 1 | 0 | 30 | 0 | - | - | 2 | 100 | 36 | 100 | 11 | 100 |
| F1 (NK-377 x NK-420) | - | - | 30 | 0 | - | - | - | - | 45 | 100 | - | - |

(continued)

Flowering properties shown in summer cultivation test

| Sugar beet strain name | Outdoor field (natural day length) | | | | | | Glass greenhouse (whole-day lighting) | | | | | |
|---|---|---|---|---|---|---|---|---|---|---|---|---|
| | 1st year | | 2nd year | | 3rd year | | 1st year | | 2nd year | | 3rd year | |
| | N | Bolt (%) | N | Bolt (%) | N | Bolt (%) | N | Bolt (%) | N | Bolt (%) | N | Bolt (%) |
| F1 (NK-377 x NK-422) | - | - | 30 | 0 | - | - | - | - | 45 | 84 | - | - |
| F2 (NK-310 x NK-420) | - | - | - | - | 120 | 0 | - | - | - | - | 139 | 83 |
| F2 (NK-310 x NK-422) | - | - | - | - | 120 | 0 | - | - | - | - | 149 | 98 |
| BC1 (NK-377 x NK-420 x NK-310) | - | - | - | - | 120 | 0 | - | - | - | - | 149 | 55 |
| BC1 (NK-377 x NK-422 x NK-310) | - | - | - | - | 85 | 0 | - | - | - | - | 147 | 48 |
| Annual strain (type strain) | 30 | 100 | 30 | 100 | 15 | 100 | 39 | 100 | 36 | 100 | 24 | 100 |
| Biennial strain (type strain) | 39 | 0 | 30 | 0 | 38 | 0 | 40 | 0 | 18 | 0 | 18 | 0 |
| F1 strain (type strain) | - | - | 12 | 100 | - | - | - | - | 16 | 100 | - | - |
| BC1 (biennial x annual x biennial, type strain) | - | - | - | - | 59 | 44 | - | - | - | - | - | - |

N: Number of tested plant individuals Bolt (%): Bolting rate (%) NK-420, NK-422: BLOND strain Annual strain (type strain): TA-33BB-O Biennial strain (type strain): TK 76-46/2mm-O (1st year), Monohikari (2nd year), NK-377mm-O (3rd year) F1 (type strain): NK-377mm-CMS (biennial) x TA-33BB-O (annual) BC1 (type strain): NK-377mm-CMS x TA-33B-O x NK-310mm-O (biennial)

[Example 2]

Winter cultivation test (November to February)

The sugar beet strains as used in the summer cultivation test of Example 1 were cultivated in a glass greenhouse at Hokkaido Agricultural Research Center, National Agriculture and Food Research Organization. The winter cultivation tests of the first year and of the second year were initiated on the same years of the summer cultivation tests of the first year and of the second year of Example 1.

In the test on the first year, sugar beets were sowed on November 5. After sowing, the raising of seedlings was managed in a glass greenhouse under whole-day lighting conditions (cultivation under natural day length with lighting at night; night lighting conditions: incandescent lamp 200 W, 300 lx, from 16:00 (4:00 p.m.) to 7:00 (7:00 a.m.) on the following morning) under temperature conditions of 20°C on average (15°C to 25°C). The seedlings were grown to have 2 to 4 leaves and then transplanted into pots (diameter: 10 cm; height: 20 cm). The date of transplanting was December 11. Thereafter, the seedlings were grown in the glass greenhouse under the same whole-day lighting conditions and temperature conditions as described above. Thereafter, the number of plant individuals that have bolted up to January 6 was counted, and the bolting rate was calculated in accordance with the equation described in Section 1. a) of Example 1 above.

In the test on the second year, sugar beets were sowed on November 4. After sowing, the raising of seedlings was managed in a glass greenhouse under whole-day lighting conditions (cultivation under natural day length with lighting at night; night lighting conditions: incandescent lamp 200 W, 300 lx, from 16:00 (4:00 p.m.) to 7:00 (7:00 a.m.)

on the following morning) under temperature conditions of 20°C on average (15°C to 25°C). The seedlings were grown to have 2 to 4 leaves and then transplanted into pots (diameter: 10 cm; height: 20 cm). The date of transplanting was December 22. Thereafter, the seedlings were grown in the glass greenhouse under the same whole-day lighting conditions and temperature conditions as described above. Thereafter, the number of plant individuals that have bolted up to January 19 was counted, and the bolting rate was calculated in accordance with the equation described in Section 1. a) of Example 1 above.

The results are shown in Table 2. As a result of the winter cultivation test, the bolting rate of the annual strain TA-33BB-O and that of the biennial strain (the first year: NK-280mm-O; the second year: NK-377mm-O) were 100%, and 0% to 7%, respectively. The bolting rate of the BLOND strain (NK-420 or NK-422) was 95% to 100%, which was approximately the same as the bolting rate of the annual strain. It was shown that the BLOND strains are induced to flower, without vernalization, under whole-day lighting conditions regardless of the cultivation season, as with the annual strain.

[Table 2]
Flowering properties shown in winter cultivation test

| Sugar beet strain name | Glass greenhouse (whole-day lighting) | | | |
|---|---|---|---|---|
| | 1st year | | 2nd year | |
| | N | Bolt (%) | N | Bolt (%) |
| NK-420 | 6 | 100 | 27 | 100 |
| NK-422 | 22 | 95 | 27 | 100 |
| F2 (NK-310 x NK-420) | - | - | 270 | 63 |
| F2 (NK-310 x NK-422) | - | - | 261 | 74 |
| BC1 (NK-377 x NK-420 x NK-310) | - | - | 90 | 58 |
| BC1 (NK-377 x NK-422 x NK-310) | - | - | 90 | 58 |
| Annual strain (type strain) | 18 | 100 | 27 | 100 |
| Biennial strain (type strain) | 18 | 0 | 27 | 7 |
| BC1 (biennial x annual x biennial, type strain) | - | - | 90 | 74 |

N: Number of tested plant individuals
Bolt (%): Bolting rate (%)
NK-420, NK-422: BLOND strain
Annual strain (type strain): TA-33BB-O
Biennial strain (type strain): NK-280mm-O (1st year), NK-377mm-O (2nd year)
BC1 (type strain): NK-377mm-CMS x TA-33B-O x NK-310mm-O (biennial)

Table 3 shows the number of days up to bolting observed in the glass greenhouse during the summer cultivation test and the winter cultivation test. The number of days up to bolting for the BLOND strains and the F1 strains were similar to those for the annual strain.

$$\text{The number of days up to bolting} = \text{the total number of days from sowing to the initiation of bolting for strains that have bolted} / \text{the total number of strains that have bolted}$$

It was shown that the BLOND strains are induced to flower within the number of days approximately equivalent to the number of days for the annual strain, under whole-day lighting conditions regardless of the cultivation season.

[Table 3]
Number of days up to bolting in cultivation in glass greenhouse (2nd year)

| Sugar beet strain name | Number of days up to bolting (days) | |
|---|---|---|
| | Summer | Winter |
| NK-420 | <36.0 | 48.8 |
| NK-422 | 36.5 | 55.8 |

(continued)

Number of days up to bolting in cultivation in glass greenhouse (2nd year)

| Sugar beet strain name | Number of days up to bolting (days) | |
|---|---|---|
| | Summer | Winter |
| F1 (NK-377 x NK-420) | 37.3 | - |
| F1 (NK-377 x NK-422) | 42.9 | - |
| Annual strain (type strain) | <36.0 | 51.9 |
| F1 strain (type strain) | 36.6 | - |
| Biennial strain (type strain) | - | - |

The results of Examples 1 and 2 show that the BLOND strains are less likely to bolt (i.e., less likely to be induced to flower) outdoors under natural day length conditions, but are induced to flower without vernalization treatment (chilling treatment) in a greenhouse under whole-day lighting conditions. Hence, the BLOND strains have distinctly different flowering properties under natural day length conditions and under whole-day lighting conditions from those of conventional sugar beet annual strains or biennial strains, and thus the BLOND strain is day length-sensitive (Table 4). The BLOND strains according to the present invention can be cultivated without flowering under natural day length conditions of an area where sugar beets can be cultivated outdoors. On the other hand, the BLOND strains can be induced to flower without vernalization treatment when cultivated under whole-day lighting conditions, and therefore a duration required for a single process of seed harvesting can be shortened to approximately 4 months, which is 1/3 of the conventional duration (i.e., approximately a year).

[Table 4]

| | Flowering properties without vernalization | |
|---|---|---|
| Sugar beet type | Natural day length conditions (outdoor cultivation) | Whole-day lighting conditions (Glass greenhouse) |
| Day length-sensitive (BLOND strain) | Not flowered | Flowered |
| Biennial strain | Not flowered | Not flowered |
| Annual strain | Flowered | Flowered |

The day length (the first year) and the monthly average temperature measured in Memuro-cho where the test field is present are as shown below.

[Table 5]
Day length and average temperature of each month of Memuro-cho

| Month | Day length | Daily average temperature (°C) | | |
|---|---|---|---|---|
| | | 1st year | 2nd year | 3rd year |
| April | 12 hrs and 44 min (April 1) | 4.7 | 5.8 | 6.4 |
| May | 14 hrs and 6 min (May 1) | 9.5 | 12.3 | 12.8 |
| June | 15 hrs and 8 min (June 1) | 15.4 | 16.7 | 14.9 |
| July | 15 hrs and 18 min (July 1) | 19.6 | 19.4 | 20.2 |
| August | 14 hrs and 31 min (August 1) | 20.2 | 19.6 | 19.6 |
| September | 13 hrs and 11 min (September 1) | 16.2 | 15.2 | 15.7 |
| October | 11 hrs and 45 min (October 1) | 10.3 | 8.1 | 8.4 |
| November | 10 hrs and 19 min (November 1) | 3.2 | 3.3 | 2.5 |
| December | 9 hrs and 17 min (December 1) | -1.8 | -5.6 | -4.1 |
| January | 9 hrs and 5 min (January 1) | -7.7 | -6.8 | - |
| February | 9 hrs and 57 min (February 1) | -6.9 | -5.4 | - |
| March | 11 hrs and 12 min (March 1) | -1.3 | 0.3 | - |

[Example 3]

The influence of the day length on flowering of the BLOND strains was further investigated. The BLOND strains (NK-420 and NK-422) and the annual strain TA-33BB-O and the biennial strain NK-310mm-O as type strains (control strains) were subjected to examination. On December 21 of the third year, sugar beets were sowed in paper pots and grown in a greenhouse set at approximately 20°C under natural day length conditions. The seedlings were grown to have 2 to 4 leaves and transplanted into jiffy pots (diameter: 10 cm) at 3 individuals per pot on January 21 of the following year. The transplanted seedlings were cultivated in an artificial climate chamber set at 20°C under 3 different day length conditions of 24-, 18-, and 14-hour day lengths, and then the bolting rates were investigated on March 5. The light source was a 100W incandescence lamp, and the lamp was provided above the pots by approximately 50 cm.

Table 6 shows the investigation results. The bolting rates of the biennial strain were 0% for all of the day lengths. While the bolting rate of the annual strain was 0% for the 14-hour day length, the bolting rates of the annual strain were 100% for the 18-hour day length and 24-hour day length. The bolting rate of the BLOND strain NK-422 was also 0% as with the biennial strain. While the bolting rate of NK-420 was 0% for the 14-hour day length as with the annual strain, the bolting rates of the annual strain were 100% for the 18-hour day length and 24-hour day length. In the cultivation with the 18-hour day length, the bolted stem length of NK-420 was 13 mm on average and bolting of NK-420 was found more likely to be delayed or inhibited, compared with the annual strain.

Thus, it was found that at least some BLOND strains are induced to flower even under a shorter day length than that of 24 hours (i.e., whole-day lighting conditions).

[Table 6]
Flowering properties depending on day length conditions

| Sugar beet strain name | Bolting rate (%) | | | Average bolted stem length (mm) | | |
| --- | --- | --- | --- | --- | --- | --- |
| | 14-hour Day length | 18-hour Day length | 24-hour Day length | 14-hour Day length | 18-hour Day length | 24-hour Day length |
| NK-420 | 0 | 100 | 100 | 0 | 13 | 400 |
| NK-422 | 0 | 0 | 0 | 0 | 0 | 0 |
| TA-33BB-O (annual) | 0 | 100 | 100 | 0 | 93 | 117 |
| NK-310mm-O (biennial) | 0 | 0 | 0 | 0 | 0 | 0 |

[Industrial Applicability]

The method of the present invention enables selection of a sugar beet strain that can be cultivated as a raw material for sugar production and of which the seeds can be harvested within a shorter period without vernalization treatment by shortening a dark period. According to the present invention, accelerated generation advancement of sugar beets can be realized, and development of plant varieties can be accelerated.

All publications, patents, and patent applications cited herein are incorporated herein by reference in their entirety.

**Claims**

1. A method for selecting sugar beets, comprising:

   cultivating sugar beet strains having a chilling requirement under light conditions with a continuous dark period of 6 hours or less per day, without vernalization treatment; and
   selecting a sugar beet strain induced to flower.

2. The method for selecting sugar beets according to claim 1 comprising:

   a) cultivating said sugar beet strains under natural long-day conditions without vernalization treatment and investigating whether or not flowering induction occurs;

b) cultivating said sugar beets under light conditions with a continuous dark period of 6 hours or less per day without vernalization treatment and investigating whether or not flowering induction occurs; and
c) selecting a sugar beet strain that is not induced to flower in the step a) but is induced to flower in the step b).

3. The method according to claim 1 or 2, wherein the light conditions with a continuous dark period of 6 hours or less per day are whole-day lighting conditions.

4. The method according to claim 2, wherein the natural long-day conditions are natural day length conditions with a natural day length of 14 to 17 hours.

5. The method according to any one of claims 1 to 4, wherein whether or not flowering induction occurs is determined based on a bolting rate.

6. The method for selecting sugar beets according to any one of claims 1 to 5, wherein the cultivation under the light conditions with a continuous dark period of 6 hours or less per day is carried out under temperature conditions of 15°C to 30°C.

7. A method for breeding sugar beets comprising crossing a sugar beet strain selected by the method according to any one of claims 1 to 6 as a parent strain with another sugar beet strain and obtaining offspring sugar beet plants capable of being induced to flower under light conditions with a continuous dark period of 6 hours or less per day without vernalization treatment.

# Fig. 1

A

Spring   Summer   Autumn   Winter   Spring   Summer   Autumn ($t$)

B

Autumn   Winter   Spring   Summer   ($t$)

C

Spring   Summer   ($t$)

# Fig. 2

A

B

**INTERNATIONAL SEARCH REPORT**

| International application No. |
|---|
| PCT/JP2018/030250 |

| A. CLASSIFICATION OF SUBJECT MATTER |
|---|
| Int.Cl.　A01H1/00(2006.01)i, A01G7/00(2006.01)i, A01G22/00(2018.01)i,<br>　　　　A01H3/02(2006.01)n |

According to International Patent Classification (IPC) or to both national classification and IPC

| B. FIELDS SEARCHED |
|---|

| Minimum documentation searched (classification system followed by classification symbols) |
|---|
| Int.Cl.　A01H1/00, A01G7/00, A01G22/00, A01H3/02 |

| Documentation searched other than minimum documentation to the extent that such documents are included in the fields searched | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

| Electronic data base consulted during the international search (name of data base and, where practicable, search terms used) |
|---|
| JSTPlus/JMEDPlus/JST7580 (JDreamIII), CAplus/MEDLINE/EMBASE/BIOSIS/WPIDS/WPIX (STN) |

| C. DOCUMENTS CONSIDERED TO BE RELEVANT | | |
|---|---|---|
| Category* | Citation of document, with indication, where appropriate, of the relevant passages | Relevant to claim No. |
| A | 田口和憲，他２名，人為春化処理による小規模世代促進方法の開発，てん菜研究会報(2004) vol. 46, pp. 23-30, ISSN: 0912-1048, page 23, abstract, pages 24-25, experiment 1-3, non-official translation (TAGUCHI, Kazunori et al., "Development of small-scale generation promoting method by artificial vernalization", Proceedings of the Sugar Beet Research Association (2004)) | 1-7 |

☒ Further documents are listed in the continuation of Box C.　　☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 05 November 2018 (05.11.2018) | 13 November 2018 (13.11.2018) |

| Name and mailing address of the ISA/<br>　Japan Patent Office<br>　3-4-3, Kasumigaseki, Chiyoda-ku,<br>　Tokyo 100-8915, Japan | Authorized officer<br><br><br>Telephone No. |
|---|---|

Form PCT/ISA/210 (second sheet) (January 2015)

## INTERNATIONAL SEARCH REPORT

| International application No. |
| --- |
| PCT/JP2018/030250 |

C (Continuation).    DOCUMENTS CONSIDERED TO BE RELEVANT

| Category* | Citation of document, with indication, where appropriate, of the relevant passages | Relevant to claim No. |
| --- | --- | --- |
| A | MUTASA-GOTTGENS, E. S., et al., "Bolting and flowering control in sugar beet: relationships and effects of gibberellin, the bolting gene B and vernalization", AoB PLANTS (2010) vol. 2010, no. plq012, pp. 1-13, ISSN: 2041-2851, page 2, left column, paragraph [0002], page 3, left column, paragraph [0002], page 4, left column, paragraph [0004] to right column, paragraph [0001], page 8 table 6, fig. 2 | 1-7 |
| A | DI JK, H. V., "Evolutionary change in flowering phenology in the iteroparous herb Beta vulgaris ssp. maritima: a search for the underlying mechanisms", Journal of Experimental Botany (2009) vol. 60, no. 11, pp. 3143-3155, ISSN: 0022-0957, page 3145, right column, paragraph [0001] to page 3146, left column, paragraph [0001], page 3145, fig. 1, page 3147, right column, paragraph [0006] to page 3148, left column, paragraph [0001], page 3148, fig. 2, page 3149, fig. 4 | 1-7 |
| A | 阿部純, 他2名,　テンサイの抽苔に関与する遺伝子モデル，てん菜研究会報(1994) vol. 36, pp. 152-157, ISSN: 0912-1048, page 152, right column, paragraph [0002], page 154, left column, paragraph [0002] to right column, paragraph [0001], non-official translation (ABE, Jun et al., "Genetic model involved in bolting of sugar beet", Proceedings of the Sugar Beet Research Association (1994)) | 1-7 |
| A | PIN, P. A., et al., "The role of a pseudo-response regulator gene in life cycle adaptation and domestication of beet", Current Biology (2012) vol. 22, pp. 1095-1101, ISSN: 0960-9822, page 1098, fig. 2, page 1099, fig. 3 | 1-7 |
| P, X | HOFT, N., et al., "Haplotype variation of flowering time genes of sugar beet and its wild relatives and the impact on life cycle regimes", Frontiers in Plant Science (2018) vol. 8, no. 2211, pp. 1-11, ISSN: 1664-462X, entire text, in particular, page 3, table 1, page 4, fig. 1 | 1-7 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010088364 A

- JP 2017159684 A

**Non-patent literature cited in the description**

- **TAGUCHI ; KAZUNORI ; OOGATA ; NAOKI ; NAKATSUKASA ; KEIJI.** Development of method for small-scale accelerated generation advancement by artificial vernalization treatment. *Proceedings of the Sugar Beet Research Association,* 2004, vol. 46, 23-30

- **ABEGG FA.** *J. Agric. Res.,* 1936, vol. 53 (7), 493-511